# EUROPEAN PATENT SPECIFICATION

(11) **EP 1 450 762 B1**
(45) Date of publication and mention of the grant of the patent: **17.01.2007**
(21) Application number: 02780231.3
(22) Date of filing: 29.10.2002
(51) Int. Cl.: A61K 9/00, A61K 47/36

(54) **NOVEL FORMULATION**
NEUE FORMULIERUNG
NOUVELLE FORMULATION

(30) Priority: 30.10.2001 SE 0103613
(43) Date of publication of application: 01.09.2004
(73) Proprietor: AstraZeneca AB, 151 85 Södertälje (SE)
(72) Inventor: ECCLESTON, Gillian, The University of Strathclyde, Glasgow, Central Scotland G1 1XQ (GB); PATERSON, Ronald, AstraZeneca R & D Charnwood, Loughborough, Leicestershire LE11 5RH (GB)
(86) International application number: PCT/SE2002/001956
(87) International publication number: WO 2003/037299

(56) References cited:
- US-A- 4 140 760
- COLLEN ET AL.: 'Development of gastroretentive dosage form' EUROPEAN JOURNAL OF PHARMACEUTICAL SCIENCES vol. 4, no. SUPPL. PG, 1996, page S182, XP002960559
- MANCINI F. ET AL.: 'Fruit-alginate interactions in novel restructured products' NAHRUNG vol. 44, no. 3, 2000, pages 152 - 157, XP002960558

## Description

The present invention relates to a novel, multiple unit, sustained release dosage form for oral administration and the preparation and use thereof.

### BACKGROUND OF THE INVENTION

Whilst being a convenient method for the delivery of therapeutic agents, oral administration can result in poor bioavailability and drug wastage. Rapid gastric emptying can result in low oral bioavailabilty especially when the administered therapeutic agent is only readily soluble in the low pH of the stomach and/or is absorbed only in a narrow region in the proximal part of the gastrointestinal tract. Further, oral administration suffers from the added problem that absorption time varies from patient to patient as gastric pH and drug dissolution time vary thus leading to unpredictable times to achieve peak plasma levels.

Optimisation of oral delivery in terms of improving oral bioavailability and consistency of absorption times and reducing drug wastage may be achieved by prolonging the time spent by the therapeutic agent in the gastric environment.

The art teaches of several methods for prolonging the gastric retention of dosage forms. One such method involves the use of floating dosage forms which remain buoyant on the gastric fluids by virtue of their low density. Like other gastric retention dosage forms they provide for better dissolution of the therapeutic agent in the stomach, better absorption at the intestinal site of absorption and allow topical treatment of the gastrointestinal tract as well as systemic treatment of diseases.

Floating dosage forms are commercially available as single unit and multiple unit floating systems. Madopar (Roche Products, Welwyn Garden City, UK) is an example of a single unit system and comprises levodopa and benserazide as a hydrodynamically balanced system formulation which allows plasma levels of levodopa to remain consistent.
However single unit floating systems are subject to "all or nothing" gastric emptying, a problem which can fortunately be avoided through the use of multiple unit dosage forms. In addition, multiple units provide for a more uniform distribution of the therapeutic agent through the gastrointestinal tract.

US 4140760 discloses mixed gel sytems of alginate and pectin. Whitehead *et al.* (*Eur. J. Pharm. Sci.* **4 (Supp):** S182 and *J. Controlled. Release.* 55 3-12) have designed multiple unit floating dosage forms based on calcium alginate beads. These heads, when freeze-dried during the manufacturing process, have been found to float on gastric fluids by virtue of the porous system created on freeze-drying. They can thus be retained in the stomach for more than 5.5 hours. Manufacture of the beads involves dropping sodium alginate into a solution or suspension of calcium chloride and a therapeutic agent. This causes precipitation of calcium alginate beads containing the therapeutic agent, which can then be freeze dried to form a floating dosage form.
However the range of therapeutic agents that can incorporated into these beads is limited by the fact that the calcium ions of this formulation can interact with certain pharmaceutical ingredients to reduce their absorption by the body. It would therefore be beneficial to develop a dosage form with a composition which did not comprise calcium ions such that pharmaceutical ingredients not compatible with calcium ions could be incorporated.

In accordance with the present invention there is therefore provided a multiple unit floating dosage form comprising an alginic acid salt, pectin and a pharmaceutically active ingredient where the pharmaceutically active ingredient can be compatible or incompatible with calcium ions.

### SUMMARY OF THE INVENTION

A multiple unit floating dosage form comprising an alginic acid salt selected from sodium alginate or potassium alginate, a high ester pectin containing an ester content of greater than 50% along the biopolymer chain, and a pharmaceutically active ingredient for oral administration is provided.

A process for the preparation of a multiple unit floating dosage form comprising an alginic acid salt, pectin and a pharmaceutically active ingredient is disclosed which process includes the dropwise addition of a biopolymer solution or suspension of an alginic acid salt and pectin also containing a soluble or insoluble therapeutic agent into acid; separation of the resulting hydrogel beads from the medium; snap freezing in liquid nitrogen; and freeze-drying.

A multiple unit floating dosage form comprising an alginic acid salt, pectin, and a pharmaceutically active ingredient for use in therapy is provided.

### DETAILED DESCRIPTION OF THE INVENTION

According to the present invention there is provided a multiple unit floating dosage form comprising an alginic acid salt selected from sodium alginate or potassium alginate, a high ester pectin containing an ester content of greater than 50% along the biopolymer chain, pectin and a pharmaceutically active ingredient suitable for oral administration.

Whitehead *et al.* disclose multiple unit floating dosage forms based on calcium alginate beads that can be retained in the stomach for more than 5.5 hours. However the use of these beads is limited by the fact that many pharmaceutically active ingredients are not compatible with calcium ions.

According to D. Thom *et al. (Prog. Fd. Nutr. Sci,* **6**, 97-108) biopolymer solutions of alginates and pectins will form firm resilient gels, in the absence of calcium or high concentrations of sugar, under conditions of low pH.

We have surprising found that multiple unit floating dosage forms can be manufactured from biopolymer solutions of alginates and pectins into which can also be incorporated a pharmaceutically active ingredient that will be compatible with a wider range of pharmaceutically active ingredients than those previously disclosed in the art. In particular the present invention shall be useful for the oral administration of pharmaceutically active ingredients that are not compatible with calcium or calcium ions. In addition, the dosage forms of the present invention have prolonged floatation and a consistent release profile for pharmaceutically active ingredients over a wide range of pH (pH 1 to 5). This pH range covers the pH range expected in both the fed and fasted states of the human stomach.

Further to this, drug release times for the dosage forms of the present invention can be varied by varying the degree of association between the alginic acid salt and pectin within the hydrogel system. This can be achieved by varying the ratio of alginic acid salt to pectin in the formulation or by varying the pH at which the biopolymers are acidified to form hydrogels.

The invention therefore provides for floating multiple unit dosage forms comprising an alginic acid salt, pectin and a pharmaceutically active ingredient.

The pectin is high ester pectin containing an ester content of greater than 50% along the biopolymer chains. Preferably, pectin is high methoxy pectin.

The alginic acid salt used is selected from potassium or sodium alginate. Preferably, sodium alginate is used.

The pharmaceutically active ingredient can be selected from a wide range of drugs, and may include H₂ antagonists, antibiotic, antibacterial and antifugal agents.

Examples of pharmaceutically active ingredients which have a reduced absorption in the body on interaction with calcium ions are biphosphonates such as aldendronic acid and disodium etidronate and antibacterials such as ciprofloxacin and tetracyclines. Tetracyclines include doxycycline and oxytetracycline. Consequently the present invention will be useful for the oral administration of these drugs.

Further the present invention will also be useful for increasing the bioavailability of drugs with narrow absorption windows in the upper small intestine such as frusemide, cyclosporin, allopurinol and ciprofloxacin and drugs that are poorly soluble in or not suited to the higher pH of the small intestine. Such drugs also include diazepam, chlordiazepoxide, cinnarizine and captopril.

In a preferred embodiment, the dosage form is manufactured from a biopolymer solution or suspension in which sodium alginate is present at a concentration of 1% w/v and high methoxy pectin at 1 % w/v. The solution or suspension also contains the pharmaceutically active ingredient at in an appropriate amount.

The invention further provides for a process for the manufacture of the multiple unit dosage form.

To an aqueous solution of alginic acid salt and pectin, at the appropriate concentrations, is added the required amount of a pharmaceutically active ingredient. The pharmaceutically active ingredient may be soluble or insoluble. The resulting solution or suspension is then added dropwise to acid to yield hydrogel beads that are separated from the medium, frozen and freeze-dried. The material can be frozen using commonly known techniques for freeze drying including conventional freeze drying, preferably the material is frozen by snap-freezing in liquid nitrogen. The resulting beads are loaded with the pharmaceutically active ingredient. Suitably the bead size is from about 0.1 to about 10mm, preferably the bead size is about 2.5mm.

The beads can range in size from about 1 to about 8 mm in diameter. Beads with a diameter of about 3 to about 4mm are preferred and most preferred are beads with a diameter of about 3.15mm

Preferably, the alginic acid salt used in the process is selected from potassium of sodium alginate. More preferably, sodium alginate is used.

Preferably, pectin is selected from high ester pectins containing an ester content of greater than 50 % along the biopolymer chains. More preferably high methoxy pectin is used.

The hydrogel beads can be snap frozen by means known in the art, for example by submerging them in liquid nitrogen.

The conditions for freeze-drying may, for example, be a temperature of -40°C for 24 hours.

In a preferred embodiment, the biopolymer solution or suspension to be acidified and form a hydrogel substance is such that sodium alginate is present at a concentration of 1% w/v and the high methoxy pectin at 1% w/v. The solution or suspension also contains the pharmaceutically active ingredient at in an appropriate amount.

The dosage form of the present invention is also useful for the topical treatment of the gastrointestinal tract. In particular the dosage form may be useful for the delivery of pharmaceutically active ingredients to the upper gastrointestinal mucosa to treat, for example, helicobacter pylori. Further, the present invention also allows the systemic treatment of disease. There is therefore provided the dosage form of the present invention for use in therapy and a method of treating a patient in need of therapy, comprising administering to said patient a dosage form of the present invention containing an appropriate therapeutic agent.

### EXAMPLES

### Example 1

Sodium alginate (1% w/v) and high ester pectin containing a methyl ester content of greater than 50% were dissolved in distilled water. The insoluble compound griseofulvin was suspended in the biopolymer solution. The suspension was then added drop-wise from a syringe (1 inch from the surface) with a 21-G needle attachment, to 500ml of HCl (vortex mixed) across a range of concentrations from 0.01M to 0.1M. Drug loaded hydrogel beads formed. The beads were separated from the medium, snap frozen in liquid nitrogen and freeze-dried at -40°C for 24 hours. The loading efficiency of griseofulvin was 97%.

### Example 2

Sodium alginate (1% w/v) and high ester pectin containing a methyl ester content of greater than 50% were dissolved in distilled water. The soluble compound paracetamol was dissolved in the biopolymer solution. The suspension was then added drop-wise from a syringe (1 inch from the surface) with a 21-G needle attachment, to 500ml of HCl (vortex mixed) across a range of concentrations from 0.01M to 0.1M. Drug loaded hydrogel beads formed. The beads were separated from the medium, snap frozen in liquid nitrogen and freeze-dried at -40°C for 24 hours. The loading efficiency of paracetamol was 40%.

### Example 3

The freeze-dried beads of example 1 were tested for prolonged floatation over a 12 hour period in vitro on distilled water and on simulated gastric fluid with pH ranging for pH 1 to pH 5. Floatation was independent of pH.

The drug release profile was also recorded and found to be consistent over this in vitro pH range.

## Claims

1. A multiple unit floating dosage form comprising an alginic acid salt selected from sodium alginate or potassium alginate, a high ester pectin containing an ester content of greater than 50 % along the biopolymer chains, and a pharmaceutically active ingredient.

2. A dosage form according to claim 1 wherein pectin is high methoxy pectin.

3. A dosage form according to claim 1 or 2 wherein the dosage form is manufactured from a solution or suspension comprising high methoxy pectin at a concentration ranging from 0.5 to 5 % w/v, preferably 0.5 to 1.5 % w/v.

4. A dosage form according to claim 3 wherein the high methoxy pectin is present at a concentration of 1% w/v.

5. A dosage form according to any one of claims 2 to 4 wherein the alginic acid salt is sodium alginate.

6. A dosage form according to claim 5 wherein the dosage form is manufactured from a solution or suspension comprising sodium alginate at a concentration ranging from 0.5 to 5 % w/v, preferably 0.5 to 1.5 % w/v.

7. A dosage form according to claim 5 or 6 wherein sodium alginate is present at a concentration of 1 % w/v.

8. A dosage form according to any one of claims 1 to 7 wherein the pharmaceutically active ingredient is selected from H₂ antagonists, antibiotic, antibacterial and antifugal agents.

9. A dosage form according to any one of claims 1 to 7 wherein the pharmaceutically active ingredient is selected from:
• active ingredients which have a reduced absorption in the body on interaction with calcium ions;
• active ingredients with narrow absorption windows in the upper small intestine;
• active ingredients that are poorly soluble in or not suited to the higher pH of the small intestine.

10. A dosage form according to anyone or 1 to 7 wherein the pharmaceutically active ingredient is selected from biphosphonates such as aldendronic acid and disodium etidronate, tetracyclines such as doxycycline and oxytetracycline, frusemide, cyclosporin, allopurinol, ciprofloxacin, diazepam, chlordiazepoxide, cinnarizine and captopril.

11. A dosage form according to any one of claims 1 to 10 wherein the multiple unit comprises beads.

12. A dosage form according to claim 1 wherein the beads have a diameter ranging from about 1 mm to about 8 mm.

13. A dosage form according to claim 11 wherein the beads have a diameter of about 3.15mm

14. A process for the manufacture of a dosage form according to any one of claims to 13 comprising:
• adding the required amount of a pharmaceutically active ingredient to an aqueous solution of alginic acid salt and pectin at the appropriate concentrations;
• dropwise addition of the resuming solution or suspension to acid;
• separation of the resulting hydrogel beads from the medium; and
• snap-freezing and freeze drying of the hydrogel beads.

15. A dosage form according to any one of claims 1 to 13 for use in therapy.

## Patentansprüche

1. Mehrfacheinheits-Schwimmdosierungsform, enthaltend ein Algensäuresalz ausgewählt aus Natriumalginat oder Kaliumalginat, ein Pektin mit hohem Estergehalt mit einem Estergehalt von mehr als 50% entlang der Biopolymerketten und einen pharmazeutischen Wirkstoff.

2. Dosierungsform nach Anspruch 1, wobei es sich bei dem Pektin um Pektin mit hohem Methoxygehalt handelt.

3. Dosierungsform nach Anspruch 1 oder 2, wobei die Dosierungsform aus einer Lösung oder Suspension hergestellt wird, die Pektin mit hohem Methoxygehalt in einer Konzentration im Bereich von 0,5 bis 5% w/v, vorzugsweise 0,5 bis 1,5% w/v, enthält.

4. Dosierungsform nach Anspruch 3, wobei das Pektin mit hohem Methoxygehalt in einer Konzentration von 1% w/v vorliegt.

5. Dosierungsform nach einem der Ansprüche 2 bis 4, wobei es sich bei dem Algensäuresalz um Natriumalginat handelt.

6. Dosierungsform nach Anspruch 5, wobei die Dosierungsform aus einer Lösung oder Suspension hergestellt wird, die Natriumalginat in einer Konzentration im Bereich von 0,5 bis 5% w/v, vorzugsweise 0,5 bis 1,5% w/v, enthält.

7. Dosierungsform nach Anspruch 5 oder 6, wobei das Natriumalginat in einer Konzentration von 1% w/v vorliegt.

8. Dosierungsform nach einem der Ansprüche 1 bis 7, wobei der pharmazeutische Wirkstoff ausgewählt ist aus H₂-Antagonisten, Antibiotika, antibakteriellen Mitteln und Antipilzmitteln.

9. Dosierungsform nach einem der Ansprüche 1 bis 7, wobei der pharmazeutische Wirkstoff ausgewählt ist aus:
• Wirkstoffen, die bei einer Wechselwirkung mit Calciumionen vom Körper weniger gut resorbiert werden;
• Wirkstoffe mit einem engen Resorptionsbereich im oberen Dünndarm;
• Wirkstoffe, die bei dem höheren pH-Wert des Dünndarms schlecht löslich sind, bzw. dafür nicht geeignet sind.

10. Dosierungsform nach einem der Ansprüche 1 bis 7, wobei der pharmazeutische Wirkstoff ausgewählt ist aus Biphosphonaten wie Aldendronsäure und Dinatriumetidronat, Tetracyclinen wie Doxycyclin und Oxytetracyclin, Frusemid, Cyclosporin, Allupurinol, Ciprofloxacin, Diazepam, Chlordiazepoxid, Cinnarizin und Captopril.

11. Dosierungsform nach einem der Ansprüche 1 bis 10, wobei die Mehrfacheinheit Perlen enthält.

12. Dosierungsform nach Anspruch 11, wobei die Perlen einen Durchmesser im Bereich von etwa 1 mm bis etwa 8 mm haben.

13. Dosierungsform nach Anspruch 11, wobei die Perlen einen Durchmesser von etwa 3,15 mm haben.

14. Verfahren zur Herstellung einer Dosierungsform nach einem der Ansprüche 1 bis 13, bei dem man:
• die erforderliche Menge eines pharmazeutischen Wirkstoffs zu einer wässrigen Lösung von Algensäuresalz und Pektin in den entsprechenden Konzentrationen gibt;
• die auf diese Weise erhaltene Lösung oder Suspension zu Säure tropft;
• die auf diese Weise erhaltenen Hydrogelperlen vom Medium abtrennt und
• die Hydrogelperlen schockgefriert und gefriertrocknet.

15. Dosierungsform nach einem der Ansprüche 1 bis 13 zur Verwendung in der Therapie.

## Revendications

1. Forme de dosage flottante à unités multiples comprenant un sel d'acide alginique choisi parmi l'alginate de sodium ou l'alginate de potassium, de la pectine à teneur élevée en ester, présentant une teneur en ester supérieure à 50% le long des chaînes du biopolymère, et un ingrédient pharmaceutiquement actif.

2. Forme de dosage selon la revendication 1, dans laquelle la pectine est une pectine à teneur élevée en groupes méthoxy.

3. Forme de dosage selon la revendication 1 ou 2, dans laquelle la forme de dosage est préparée à partir d'une solution ou d'une suspension constituée par de la pectine à teneur élevée en groupes méthoxy en une concentration allant de 0,5 à 5% P/V, de préférence de 0,5 à 1,5% P/V.

4. Forme de dosage selon la revendication 3, dans laquelle la pectine à teneur élevée en groupes méthoxy est présente en une concentration de 1% P/V.

5. Forme de dosage selon l'une quelconque des revendications 2 à 4, dans laquelle le sel d'acide alginique est l'alginate de sodium.

6. Forme de dosage selon la revendication 5, dans laquelle la forme de dosage est préparée à partir d'une solution ou d'une suspension constituée par de l'alginate de sodium en une concentration allant de 0,5% à 5% P/V, de préférence de 0,5% à 1,5% P/V.

7. Forme de dosage selon la selon la revendication 5 ou 6, dans laquelle l'alginate de sodium est présent en une concentration de 1% P/V.

8. Forme de dosage selon l'une quelconque des revendications 1 à 7, dans laquelle l'ingrédient pharmaceutiquement actif est choisi parmi les agents antagonistes de H₂, les agents antibiotiques, antibactériens et antifongiques.

9. Forme de dosage selon l'une quelconque des revendications 1 à 7, dans laquelle l'ingrédient pharmaceutiquement actif est choisi parmi :
- les ingrédients actifs qui présentent une absorption réduite dans le corps suite à une interaction avec les ions de calcium ;
- les ingrédients actifs présentant des fenêtres d'absorption étroites dans l'intestin grêle supérieur ;
- les ingrédients actifs qui sont peu solubles dans ou ne conviennent pas au pH élevé de l'intestin grêle.

10. Forme de dosage selon l'une quelconque des revendications 1 à 7, dans laquelle l'ingrédient pharmaceutiquement actif est choisi parmi les diphosphonates tels que l'acide aldendronique et l'étidronate disodique, les tétracyclines telles que la doxycycline et l'oxytétracycline, le frusémide, la cyclosporine, l'allopurinol, la ciprofloxacine, le diazépam, le chlorodiazépoxyde, la cinnarizine et le captopril.

11. Forme de dosage selon l'une quelconque des revendications 1 à 10, dans laquelle l'unité multiple est constituée par des perles.

12. Forme de dosage selon la revendication 11, dans laquelle les perles présentent un diamètre allant d'environ 1 mm à environ 8 mm.

13. Forme de dosage selon la revendication 11, dans laquelle les perles présentent un diamètre d'environ 3,15 mm.

14. Procédé pour la préparation d'une forme de dosage selon l'une quelconque des revendications 1 à 13 constitué par :
- l'addition de la quantité nécessaire d'un ingrédient pharmaceutiquement actif à une solution aqueuse de sel d'acide alginique et de pectine aux concentrations élevées ;
- addition goutte à goutte de la solution ou de la suspension obtenue à un acide ;
- séparation des perles d'hydrogel obtenues du milieu ; et
- congélation immédiate et lyophilisation des perles d'hydrogel.

15. Forme de dosage selon l'une quelconque des revendications 1 à 13 destinée à une utilisation en thérapie.
